# EUROPÄISCHE PATENTSCHRIFT

(11) **EP 0 761 635 B1**
(45) Veröffentlichungstag und Bekanntmachung des Hinweises auf die Patenterteilung: **04.08.1999**
(21) Anmeldenummer: 96113605.8
(22) Anmeldetag: 24.08.1996
(51) Int. Cl.: C07C 45/50, C07C 67/313, C07C 47/02, C07C 69/73

(54) **Verfahren zur Hydroformylierung olefinisch ungesättigter Verbindungen**
Process for the hydroformylation of olefinically unsaturated compounds
Procédé d'hydroformylation de composés à insaturation oléfinique

(30) Priorität: 02.09.1995 DE 19532393
(43) Veröffentlichungstag der Anmeldung: 12.03.1997
(73) Patentinhaber: Celanese GmbH, 60439 Frankfurt (DE)
(72) Erfinder: Bahrmann, Helmut, Dr., 46499 Hamminkeln (DE); Lappe, Peter, Dr., 46539 Dinslaken (DE); Fell, Bernhard, Prof. Dr., 52074 Aachen (DE); Xia, Zhigao, M. Sc, CH-4334 Sisseln (CH); Kanagasabapathy, Subba, N.C.L. Colony, Pune - 411 008 (IN)

(56) Entgegenhaltungen:
- EP-A- 0 268 268
- EP-A- 0 407 687
- DE-A- 2 627 354
- US-A- 4 808 756
- PATENT ABSTRACTS OF JAPAN vol. 008, no. 062 (C-215), 23.März 1984 & JP-A-58 216138 (KURARAY KK), 15.Dezember 1983,

## Beschreibung

Die Erfindung betrifft ein Verfahren zur Hydroformylierung olefinisch ungesättigter Verbindungen, deren Hydroformylierungsprodukte in Wasser nicht löslich sind. Die Umsetzung erfolgt in homogener Phase in einem polaren organischen Lösungsmittel, bei dem es sich um einen niedermolekularen aliphatischen Alkohol mit 1 bis 4 Kohlenstoffatomen im Molekül oder um ein Gemisch aus zwei oder mehreren dieser Alkohole handelt, als Reaktionsmedium. Das Katalysatorsystem, es besteht aus einer Rhodiumcarbonylverbindung und dem Salz eines sulfonierten oder carboxylierten organischen Mono- oder Polyphosphins, ist sowohl in dem polaren organischen Lösungsmittel als auch in Wasser löslich.

Für die Hydroformylierung olefinisch ungesättigter Verbindungen haben sich in den letzten Jahren zwei Verfahren durchgesetzt. Das eine arbeitet in homogener Phase, d.h. Ausgangsolefin, Katalysatorsystem (Rhodiumcarbonyl und organisches Phosphin) und Reaktionsprodukte liegen gemeinsam als Lösung vor. Die Reaktionsprodukte werden aus dem Reaktionsgemisch destillativ abgetrennt.

So offenbart EP-A-0 268 268 ein homogenes nichtwäßriges Hydroformylierungsverfahren zur Hydroformylierung von olefinisch ungesättigten Verbindungen, die 2 bis 5 Kohlenstoffatome im Molekül oder 6 bis 20 Kohlenstoffatome im Molekül aufweisen, in Gegenwart eines polaren organischen Lösungsmittels. Als polare organische Lösungsmittel werden hochmolekulare Verbindungen, wie z.B. Polyalkylenglykole, Sulfolan oder Dimethylsulfoxid, aber auch niedermolekulare polare Lösungsmittel, wie z.B. Methanol oder Ethanol, verwendet. Nach dem aus EP-A-0 268 268 bekannten Prozeß wird der gewünschte Aldehyd aus dem Reaktionsgemisch abdestilliert, während im Destillationsrückstand das Katalysatorsystem und das hochmolekulare polare Lösungsmittel zurückbleibt.

Der andere Prozeß ist durch das Vorhandensein einer vom Reaktionsprodukt getrennten wäßrigen Katalystorphase, die Rhodiumcarbonyl und ein sulfoniertes oder carboxyliertes organisches Phosphin enthält, charakterisiert. Diese Variante der Umsetzung erlaubt die Isolierung der Hydroformylierungsprodukte ohne Anwendung thermischer Verfahrensschritte, sie vereinfacht die Rückführung des Katalysators und ergibt einen besonders hohen Anteil unverzweigter Aldehyde bei Einsatz endständiger Olefine.

Die Hydroformylierung höherer, einfach oder mehrfach olefinisch ungesättigter Verbindungen findet zunehmendes Interesse. Es erstreckt sich nicht nur auf die Umsetzung von Kohlenwasserstoffen, sondern auch auf Verbindungen, die neben Doppelbindungen noch weitere reaktive funktionelle Gruppen enthalten. Beispielhaft für solche Verbindungsklassen mit technischer Bedeutung sind die ungesättigten Fettsäureester. Sie haben häufig nativen Ursprung oder werden aus nativen Rohstoffen hergestellt und stehen in großen Mengen zur Verfügung. Die Reaktionsprodukte der Hydroformylierung, Mono- oder Polyformylcarbonsäureester, die auch noch nicht umgesetzte Doppelbindungen enthalten können, sind gesuchte Zwischenprodukte, die zu vielfach genutzten Erzeugnissen wie Polyaminen, Polyurethanen, Alkydharzen, Weichmachern und synthetischen Schmierstoffen weiterverarbeitet werden.

Die Hydroformylierung höherer, einfach oder mehrfach olefinisch ungesättigter Verbindungen nach dem Homogenverfahren mit Rhodiumcarbonyl/Phosphin-Katalysatoren ist wiederholt untersucht worden. Die Wirtschaftlichkeit dieses Verfahrens ist nur dann gewährleistet, wenn es gelingt, das homogen gelöste Katalysatorsystem von den Reaktionsprodukten verlustfrei abzutrennen und in aktiver Form in den Hydroformylierungsreaktor zurückzuführen. Bisher ist es lediglich möglich, den Katalysator aus Formylfettsäureester enthaltenden Reaktionsgemischen der Hydroformylierung einfach ungesättigter Fettsäureester zu entfernen. Die Arbeitsweise erfordert jedoch aufwendige Maßnahmen, überdies fällt der Katalysator in inaktiver Form an und der Phosphinanteil des Katalysatorsystems geht vollständig verloren (J.Amer. Oil Chem. Soc. Vol. 50, 455 (1973)).

Bei Einsatz mehrfach ungesättigter Verbindungen mit isolierten, aber nahe beieinanderliegenden Doppelbindungen gelingt zwar die Hydroformylierung unter Vermeidung einer Doppelbindungsisomerisierung, die Abtrennung und Rückführung des im Reaktionsprodukt homogen gelösten Katalysators, z.B. durch Destillation, ist aber nicht möglich.

Nach der aus JP-A-58216138 bekannten Arbeitsweise erfolgt die Hydroformylierungsreaktion von 1,5-Hexadien oder 1,7-Octadien in Gegenwart eines Gemisches aus Wasser und einem polaren Lösungsmittel, wie z.B. Sulfolan oder 1,4-Butandiol. Die Abtrennung des gewünschten Hydroformylierungsproduktes aus dem Reaktionsgemisch geschieht durch Extraktion mit einem gesättigten aliphatischen Alkohol mit 5 bis 12 Kohlenstoffatomen im Molekül oder mit einer Mischung aus diesen Alkoholen mit gesättigten aliphatischen oder alicyclischen Kohlenwasserstoffen, die im Molekül 5 bis 10 Kohlenstoffatome besitzen. Die nach der Extraktion verbleibende Katalysatorphase wird anschließend in den Hydroformylierungsprozeß zurückgeführt.

Nach US-A-4,808,756 lassen sich α,ω-Dialdehyde aus α,ω-Diolefinen oder α,ω-Alkenalen durch Hydroformylierung in einem Sulfolan/Wasser-Gemisch erhalten. Das gewünschte Hydroformylierungsprodukt gewinnt man durch Extraktion mit einem gesättigten alicyclischen Kohlenwasserstoff mit 5 bis 10 Kohlenstoffatomen im Molekül.

Linol- und Linolensäuremethylester können in Gegenwart heterogenisierter Rhodiumcarbonyl/Phosphin-Komplexkatalysatoren auf Polysiloxanbasis hydroformyliert werden (Chemikerzeitung, 115 (1991, S. 39 ff)). Das Verfahren liefert bei Einsatz von Linolsäuremethylester Monoformylstearylsäureester in Ausbeuten bis zu 79 %, bezogen auf den eingesetzten zwei fach ungesättigten Ester. Linolensäure ergibt bei der Hydroformylierung in Gegenwart des genannten Katalysatorsystems maximal 50 % Diformylverbindungen, Triformylprodukte werden dagegen allenfalls in untergeordneten Mengen (weniger als 10 %) erhalten. Der Rhodiumaustrag liegt im Mittel bei etwa 0,5 % des ursprünglich eingesetzten Edelmetalls. Nicht auszuschließen ist, daß ein Anteil des Katalysatormetalls im Gleichgewicht mit dem fixierten Metall homogen gelöst vorliegt, so daß die Hydroformylierung nicht nur am Festbettkatalysator, sondern auch in Lösung stattfindet.

Schwierigkeiten, Reaktionsprodukt und Katalysatorsystem voneinander zu trennen, treten bei der Hydroformylierung höherer olefinisch ungesättigter Verbindungen in Gegenwart einer wäßrigen Katalysatorlösung nicht auf. Aufgrund der geringen Löslichkeit der Olefine in Wasser befriedigt jedoch oftmals nicht ihr Umsatz. Dieser Nachteil läßt sich vermeiden, wenn man nach EP-B-157 316 die Hydroformylierung von Olefinen mit mehr als 6 Kohlenstoffatomen im Molekül in Gegenwart einer wäßrigen Lösung, die als Katalysator Rhodium-Komplexverbindungen und überdies als Lösungsvermittler ein quartäres Ammoniumsalz enthält, durchführt. Eine Weiterentwicklung dieses Prozesses ist Gegenstand der EP-B-163 234. Nach der Lehre dieser Patentschrift werden C₆- bis C₂₀-Olefine mit Wasserstoff und Kohlenmonoxid in Gegenwart von Rhodium und dem Salz eines sulfonierten Arylphosphins umgesetzt, dessen Kation ein quartäres Ammoniumion ist. Das quartäre Ammoniumsalz des Phosphins wirkt hierbei nicht nur als Katalysatorkomponente, sondern gleichzeitig auch als Lösungsvermittler. Beide Verfahren betreffen ausschließlich die Umsetzung einfach ungesättigter Verbindungen, die keine funktionellen Gruppen enthalten.

Es bestand daher die Aufgabe, ein Verfahren zu entwickeln, das es erlaubt, höhermolekulare olefinisch ungesättigte Verbindungen zu hydroformylieren, wobei mehrfach ungesättigte Ausgangsstoffe nicht nur partiell, sondern vollständig zu Formylverbindungen umgesetzt werden. Darüber hinaus sollen Reaktionsprodukt und Katalysatorsystem leicht voneinander getrennt und hierbei Edelemtallverluste weitgehend vermieden werden.

Die vorstehend beschriebene Aufgabe wird gelöst durch ein Verfahren zur Hydroformylierung olefinisch ungesättigter Verbindungen, deren Hydroformylierungsprodukte in Wasser nicht löslich sind, durch Umsetzung von olefinisch ungesättigten Verbindungen in homogener Phase in einem polaren organischen Lösungsmittel, bei dem es sich um einen niedermolekularen aliphatischen Alkohol mit 1 bis 4 Kohlenstoffatomen im Molekül oder um ein Gemisch aus zwei oder mehreren dieser Alkohole handelt und in Gegenwart eines Katalysatorsystems, das eine Rhodiumcarbonylverbindung und ein sowohl in dem polaren organischen Lösungsmittel als auch in Wasser löslichens Salz eines sulfonierten oder carboxylierten organischen Mono- oder Polyphosphins enthält, bei 60 bis 180°C und 1 bis 35 MPa mit Kohlenmonoxid und Wasserstoff. Es ist dadurch gekennnzeichnet, daß man aus dem Reaktionsgemisch das polare organische Lösungsmittel abdestilliert und aus dem Destillationsrückstand das Katalysatorsystem mit Wasser abtrennt.

Das neue Verfahren kombiniert die Eigenheiten der Hydroformylierung in homogener Phase mit den Vorteilen der Hydroformylierung in Gegenwart einer heterogenen, d.h. wäßrigen Katalysatorphase zu einer neuen, fortschrittlichen Arbeitsweise, die sich hervorragend zur Umsetzung höhermolekularer, einfach oder mehrfach olefinisch ungesättigter Verbindungen bewährt. Es wird mit besonderem Erfolg zur Hydroformylierung von Estern ungesättigter, vorzugsweise mehrfach ungesättigter Fettsäuren angewandt.

Überraschenderweise gelingt es nämlich nach dem erfindungsgemäßen Prozeß, mehrere im Estermolekül enthaltene Doppelbindungen, die auch innenständig sein können, gleichzeitig zu hydroformylieren, so daß z.B. aus zweifach ungesättigten Verbindungen Diformylprodukte und aus dreifach ungesättigten Verbindungen Triformylprodukte entstehen. Der neue Prozeß ersetzt ferner das heterogene Reaktionssystem aus olefinisch ungesättigter Verbindung und wäßriger Katalysatorlösung, das charakteristisch für die Umsetzung gemäß EP-B-167 316 und EP-B-163 234 ist, durch eine homogene Lösung von Ausgangsstoff und Katalysator mit der Folge, daß die Reaktionsgeschwindigkeit erhöht und der Umsatz verbessert wird.

Die als Einsatzstoffe für das erfindungsgemäße Verfahren geeigneten höhermolekularen olefinisch ungesättigten Verbindungen müssen in dem als Reaktionsmedium verwendeten polaren organischen Lösungsmittel löslich sein und Hydroformylierungsprodukte ergeben, die in Wasser nicht oder nur wenig löslich sind. Dementsprechend können nach dem neuen Prozeß acyclische Monoolefine mit sechs und mehr Kohlenstoffatomen im Molekül, insbesondere C₁₀- bis C₃₀-Monoolefine wie Tri- und Tetrabutene und -isobutene umgesetzt werden. Auch mehrfach ungesättigte acyclische oder cyclische Olefine mit mindestens sechs Kohlenstoffatomen im Molekül, unter ihnen insbesondere mono- und bicyclische Olefine, lassen sich nach dem erfindungsgemäßen Verfahren erfolgreich hydroformylieren. Beispielhaft für diese Verbindungsklassen sind Dicyclopentadien, Cyclooctatrien-1.5, Cyclododecatrien-1.5.9, 1.5-Hexadien, 1.7-Octadien, 1.9-Decadien. Besonders gut eignet sich die neue Arbeitsweise, wie bereits gesagt, zur Hydroformylierung ungesättigter Fettsäureester. Diese Ester leiten sich insbesondere von zweifach oder dreifach ungesättigten Fettsäuren mit 8 bis 25, vorzugsweise 10 bis 20 Kohlenstoffatomen im Molekül und von gesättigten aliphatischen Monoalkoholen mit 1 bis 10 Kohlenstoffatomen im Molekül, vorzugsweise Methanol, ab. Man erhält diese Ester aus natürlichen Ölen, die gegebenfalls zuvor raffiniert und destilliert wurden, durch Umesterung. Beispiele für natürliche Öle als Basis für die Säurekomponente der Ausgangsester sind Baumwollsaatöl, Distelöl, Erdnußöl, Kürbiskernöl, Leinöl, Maiskeimöl, Sojaöl und Sonnenblumenöl.

Als Katalysatoren werden im beanspruchten Verfahren Systeme eingesetzt, die in polaren organischen Lösungsmittel und in Wasser löslich sind und als eine Komponente eine Rhodiumcarbonylverbindung und als weitere Komponente ein sowohl in Wasser als auch im polaren organischen Lösungsmittel lösliches Salz eines sulfonierten oder carboxylierten organischen Phosphins enthalten.

Rhodiumcarbonyl und Phosphin gehen miteinander eine komplexe Bindung in der Weise ein, daß ein Teil der Kohlenmonoxidmoleküle in der Rhodiumcarbonylverbindung durch Phosphinmoleküle als Liganden ersetzt werden. Dabei wird die Löslichkeit der Rhodium/Phosphin-Komplexverbindung durch die Löslichkeit des Phosphins bestimmt. Üblicherweise liegt das Phosphin bezogen auf Rhodium im Überschuß vor, d.h. im Katalysatorsystem ist neben der Rhodium/Phosphin-Komplexverbindung auch noch freies Phosphin vorhanden.

Unter dem Begriff organische Phosphine werden Mono- oder Polyphosphine verstanden, in denen an die Valenzen des dreiwertigen Phosphoratoms oder der dreiwertigen Phosphoratome Alkyl- und/oder Arylgruppen gebunden sind, von denen mindestens eine einfach oder mehrfach sulfoniert oder carboxyliert ist. Beispiele für aliphatische Gruppen sind geradkettige oder verzweigte Reste gesättigter Kohlenwasserstoffe mit 2 bis 8 Kohlenstoffatomen und cyclischer Kohlenwasserstoffe mit 5 bis 8 Kohlenstoffatomen im Molekül. Typisch für aromatische Reste sind der Phenyl-, der Benzyl- und der Naphthylrest. Sowohl die aliphatischen als auch die aromatischen Reste können durch weitere Atomgruppierungen oder Atome wie Alkyl, Hydroxyl, Halogen substituiert sein. Die Bezeichnung organische Phosphine schließt auch solche Verbindungen des dreiwertigen Phosphors ein, in denen das Phosphoratom Bestandteil eines heterocyclischen Ringes ist.

Die im Katalysatorsystem enthaltenen Phosphine brauchen nicht einheitliche chemische Verbindungen zu sein, sondern können unterschiedliche chemische Zusammensetzung aufweisen. So können sie sich z.B. durch Art und Bindung der am Phosphoratom haftenden organischen Reste, durch den Sulfonierungs- oder Carboxylierungsgrad oder durch die Art der Kationen unterscheiden. Entscheidend für ihre Eignung als Katalysatorbestandteil ist ihre Löslichkeit in Wasser und in polaren organischen Lösungsmitteln. Dieses Kriterium erfüllen insbesondere Salze sulfonierter bzw. carboxylierter Phosphine, deren Kation Lithium oder ein Ammonium-Ion der allgemeinen Formel N(R¹R²R³R⁴)⁺ ist. R¹, R², R³ und R⁴ können gleich oder verschieden sein und stehen für Wasserstoff und für geradkettige oder verzweigte Alkylreste, insbesondere Alkylreste mit 1 bis 4 Kohlenstoffatomen.

Die Anionen der sulfonierten oder carboxylierten Monophosphine entsprechen bevorzugt der allgemeinen Formel (1) 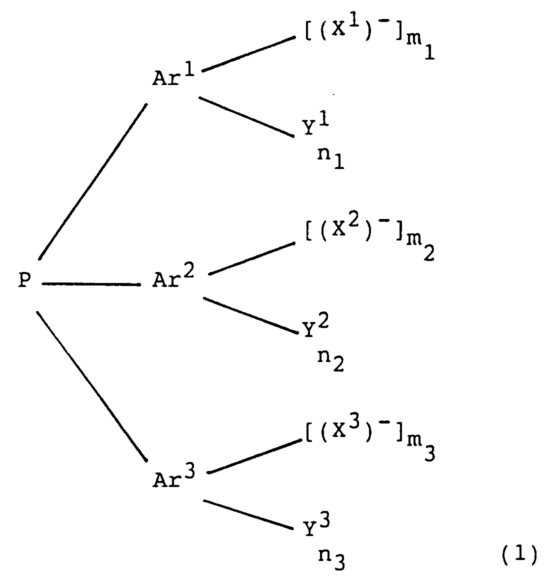 ab. Hierbei bedeuten Ar¹, Ar², Ar³ jeweils eine Phenyl- oder Naphthylgruppe, X¹, X², X³ jeweils einen Sulfonat-(-SO₃⁻) und/oder einen Carboxylat-(-COO⁻) Rest, Y¹, Y², Y³ ist jeweils eine geradkettige oder verzweigte Alkylgruppe mit 1 bis 4 C-Atomen, eine Alkoxygruppe, ein Halogenatom, die OH-, CN-, NO₂- oder die (R⁵R⁶)N-Gruppe, in der R⁵ und R⁶ für jeweils eine geradkettige oder verzweigte Alkylgruppe mit 1 bis 4 C-Atomen stehen, m₁, m₂, m₃ sind gleiche oder verschiedene ganze Zahlen von 0 bis 5 mit der Maßgabe, daß mindestens ein m₁, m₂ oder m₃ größer als 0 ist; n₁, n₂, n₃ sind gleiche oder verschiedene ganze Zahlen von 0 bis 5.

Besonders geeignete Salze leiten sich vom Anion der allgemeinen Formel (1) ab, in der Ar¹, Ar², Ar³ jeweils einen Phenylrest und X¹, X², X³ jeweils einen in meta-Stellung zum Phosphor befindlichen Sulfonatrest bedeuten, d.h. Salzen des Tris(m-sulfonatophenyl)phosphins (abgekürzt TPPTS). Weiterhin haben sich Salze des Diphenyl(m-sulfonatophenyl)-phosphins (abgekürzt TPPMS), unter ihnen insbesondere das Lithiumsalz (Li-TPPMS), als Katalysatorkomponente bewährt.

Eine weitere Gruppe als Katalysatorkomponenten bewährter Monophosphin-Anionen folgt der allgemeinen Formel (2)

A₂P - (CH₂)ₙ - CH(R) - SO₃⁻ (2)

in der A gleiche oder verschiedene Alkyl- und/oder Arylreste bedeutet, n 0, 1 oder 2 ist und R für Wasserstoff oder einen Alkylrest steht. Man erhält die Verbindungen durch Sulfalkylierung von Dialkyl- oder Diarylphosphinen mit 1,2-, 1,3- oder 1,4-Sultonen 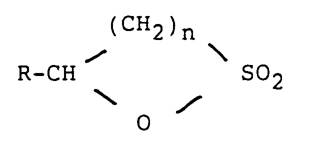 (mit n = 1, 2 oder 3 und R = H, Alkyl),
z.B. entsprechend

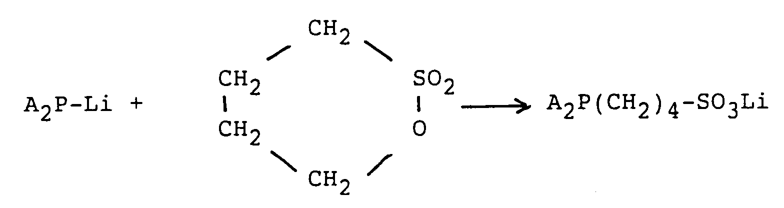

Das Alkalisalz kann nach gebräuchlichen Verfahren in ein Ammoniumsalz umgewandelt werden.

Das Anion kann außer von Monophosphinen auch von Polyphosphinen, insbesondere Diphosphinen, die mindestens einen sulfonierten oder carboxylierten organischen Rest enthalten, gebildet werden. Diphosphinanionen leiten sich bevorzugt von Diarylverbindungen der allgemeinen Formel (3) 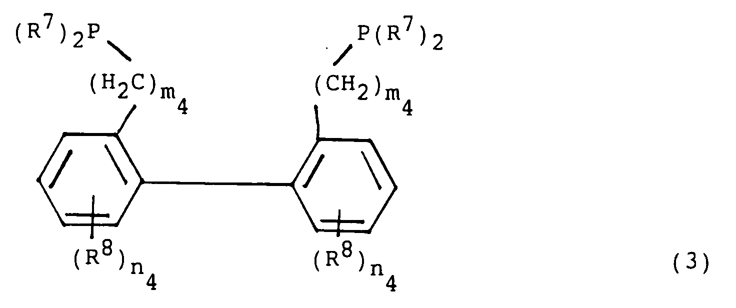 ab, die durch mindestens einen Sulfonatrest (-SO₃⁻) oder Carboxylatrest (-COO⁻) substituiert sind. In der allgemeinen Formel stehen R⁷ für gleiche oder verschiedene Alkyl-, Cycloalkyl-, Phenyl-, Tolyl- oder Naphthylreste, R⁸ ist gleich oder verschieden und bedeutet Wasserstoff, Alkyl- oder Alkoxyreste mit 1 bis 14 C-Atomen, ferner Cycloalkyl-, Aryl- oder Aroxyreste mit 6 bis 14 C-Atomen oder einen annelierten Benzolring, m₄ ist gleich oder verschieden und steht für ganze Zahlen von 0 bis 5 und n₄ ist ebenfalls gleich oder verschieden und steht für ganze Zahlen von 0 bis 4. Bevorzugt werden die sulfonierten Verbindungen; sie sind nach gängigen Methoden zugänglich. Bewährte Vertreter dieser Verbindungsklasse sind die durch Sulfonierung von 2,2'-Bis(diphenylphosphinomethyl)-1,1'-biphenyl oder 2,2'-Bis(diphenylphosphinomethyl)-1,1'-binaphthyl erhaltenen Produkte. Als Beispiel des Anions einer heterocyclischen Phosphorverbindung ist das 3,4-Dimethyl-2,5,6-tris(p-sulfonatophenyl)-1-phosphanorbornadien zu nennen.

Die Reaktion der olefinisch ungesättigten Ausgangsverbindungen mit Wasserstoff und Kohlenmonoxid erfolgt bei Temperaturen von 60 bis 180°C, insbesondere 100 bis 140°C und Drücken von 1 bis 35 MPa. Für die Hydroformylierung von Olefinen haben sich insbesondere Drücke von 2 bis 35 MPa bewährt, Ester ungesättigter Fettsäuren werden vorzugsweise bei Drücken von 15 bis 25 MPa umgesetzt.

Die Umsetzung führt man in einem polaren organischen Lösungsmittel als Reaktionsmedium durch, das sowohl die olefinisch ungesättigte Ausgangsverbindung als auch das Reaktionsprodukt und Katalysatorsystem löst. Geeignete Lösungsmittel sind niedermolekulare aliphatische Alkohole mit ein bis vier Kohlenstoffatomen im Molekül. Statt reiner Lösungsmittel können auch Gemische aus zwei oder mehreren dieser Alkohole wie Methanol/Ethanol-, Methanol/i-Propanol-Gemische verwendet werden. Besonders bewährt als Reaktionsmedium haben sich Methanol und Ethanol, die bis zu 5 Gew.-% Wasser enthalten können, jedoch vorzugsweise wasserfrei eingesetzt werden.

Der Katalysator kann dem Reaktionssystem präformiert zugesetzt werden. Mit gleich gutem Erfolg läßt er sich aber auch aus den Komponenten Rhodium oder Rhodiumverbindung und der Lösung des sulfonierten oder carboxylierten Phosphins im Reaktionsgemisch, d.h. olefinisch ungesättigte Verbindung und Lösungsmittel, unter Reaktionsbedingungen herstellen. Außer metallischem Rhodium in feinverteilter Form können als Rhodiumquelle anorganische Rhodiumsalze wie Rhodiumchlorid, Rhodiumsulfat, Rhodiumsalze organischer Säuren, wie Rhodiumacetat, Rhodium-2-ethylhexanoat oder Rhodiumoxide eingesetzt werden.

Die Rhodiumkonzentration in der Reaktionslösung beträgt 100 bis 600 Gew.-ppm, vorzugsweise 300 bis 400 Gew.-ppm, bezogen auf die Lösung. Man setzt das Phosphin in einer solchen Menge ein, daß je mol Rhodium mindestens 20 mol, vorzugsweise 40 bis 80 mol, P(III) vorliegen.

Der pH-Wert der Reaktionslösung soll einen Wert von 3 nicht unterschreiten. Allgemein stellt man einen pH-Wert von 4 bis 11 ein. Bei Verwendung von Methanol als Lösungsmittel kann eine Acetalisierung der durch die Hydroformylierung gebildeten Aldehyde eintreten. Ist sie zum Schutz der Carbonylgruppe vor Folgereaktionen erwünscht, empfiehlt es sich, bei pH-Werten von 4,5 bis 6,5, insbesondere 5,5 bis 6,0 zu arbeiten.

Das Verhältnis von Kohlenmonoxid zu Wasserstoff im Synthesegas kann in weiten Grenzen variiert werden. Im allgemeinen setzt man Synthesegas ein, in dem das Volumverhältnis von Kohlenmonoxid zu Wasserstoff 1 : 1 beträgt oder von diesem Wert nur wenig abweicht.

Die Umsetzung kann sowohl absatzweise als auch kontinuierlich durchgeführt werden.

Aus dem Reaktionsprodukt wird zunächst das polare organische Lösungsmittel abdestilliert. Anschließend wäscht man den Destillationsrückstand vorzugsweise bei Normaltemperatur mit Wasser, um das Katalysatorsystem aus den Aldehyden abzutrennen. Diese Behandlung kann gegebenenfalls mehrfach wiederholt werden. Zur Vervollständigung der Rhodiumrückgewinnung hat es sich als zweckmäßig erwiesen, dem Waschwasser ein zur Komplexbildung mit Rhodium befähigtes Phosphin, zweckmäßig ein Phosphin, das gleichzeitig Katalysatorkomponente ist, zuzusetzen.

Das erfindungsgemäße Verfahren wird durch die nachfolgenden Beispiele erläutert, jedoch nicht auf die dargestellten Ausführungsformen beschränkt.

### Beispiel 1

In ein mit Argon gespültes Schlenkrohr werden 15 ml Methanol, 60 mg (0,05 mmol) einer 8,5 Gew.-%igen Rhodiumlösung (als wäßrige Rh₂(SO₄)₃-Lösung) und 700 mg (2 mmol) Diphenyl-4-lithiumsulfonatobutyl-phosphin gefüllt. Das P/Rh-Molverhältnis beträgt 40. Diese Lösung wird auf einen pH-Wert von 5 eingestellt und dann mittels einer Spritze in einem mit Argon gespülten und gefüllten Laborautoklav vorgelegt. In den druckfesten Tropftrichter des Autoklavs werden 15 g eines 90 Gew.-%igen Linolensäuremethylesters (Rest: Linolsäuremethylester) gegeben. Der Autoklav wird mit Wassergas gespült, anschließend wird ein Reaktionsdruck von 20 MPa und eine Reaktionstemperatur von 120°C eingestellt. Nach einer Präformierzeit von einer Stunde tropft man den Linolensäuremethylester zu und verfolgt die Druckabnahme über einen Druckaufnehmer und einen Registrierschreiber. Die Gasaufnahme ist nach 10 h beendet. Der Autoklav wird abgekühlt, entspannt und sein Inhalt durch Destillation unter Luftausschluß (Argon) vom Methanol befreit. Der Rückstand wird zur Extraktion des Katalysatorsystems zweimal mit je 15 - 20 ml sauerstofffreiem Wasser gewaschen und dann analysiert. Der Umsatz an Linolen- und Linolsäureester ist quantitativ, die Ausbeuten betragen:
- 5 %: Monoformylprodukt bezogen auf das Gesamtedukt,
- 22 %: Diformylprodukt bezogen auf das Gesamtedukt und
- 82 %: Triformylprodukt bezogen auf den eingesetzten Linolensäuremethylesteranteil im Edukt.

### Beispiele 2 bis 6

In ähnlicher Weise wie in Beispiel 1 werden unter Variation des Druckes, des pH-Wertes der Katalysatorlösung und des P/Rh-Verhältnisses die Beispiele 2 bis 6 ausgeführt. Die erhaltenen Ergebnisse sind in der folgenden Tabelle zusammengestellt.

**Tabelle 1**

| Beispiel | P/Rh | pH | Temp. [°C] | Druck [MPa] | Zeit¹⁾ [h] | MF²⁾ | DF³⁾ | TF⁴⁾ | Umsatz [%] |
|---|---|---|---|---|---|---|---|---|---|
| | | | | | | [ mol-% ] | | | |
| 2 | 40 | 8.4 | 120 | 20 | 3 | 4 | 24 | 80 | 100 |
| 3 | 20 | 5.0 | 120 | 20 | 3 | 14 | 44 | 47 | 100 |
| 4 | 10 | 5.0 | 120 | 20 | 3 | 16 | 44 | 45 | 100 |
| 5 | 10 | 5.0 | 120 | 7 | 3 | 20 | 50 | 34 | 100 |
| 6 | 20 | 5.0 | 120 | 7 | 3 | 15 | 59 | 40 | 100 |

| | | | | | | | | | |
|---|---|---|---|---|---|---|---|---|---|
| 1) Zeit, nach der 90 bis 95 % des Gases aufgenommen sind | | | | | | | | | |
| 2) Monoformylprodukt, bezogen auf Gesamtedukt | | | | | | | | | |
| 3) Diformylprodukt, bezogen auf Gesamtedukt | | | | | | | | | |
| 4) Triformylprodukt, bezogen auf eingesetzten Linolensäuremethylester | | | | | | | | | |

### Beispiel 7

In einem mit Argon gespülten 100 ml-V4A-Edelstahlautoklav mit Magnetkernrührer, druckfestem Dosiergefäß, Thermoelementstutzen und Druckaufnehmer (Dehnungsmeßstreifen) werden 24 mg Rhodium (0,05 mmol) als wäßrige Rh₂(SO₄)₃-Lösung und 2,2 g (3 mmol) Tri(tetramethylammonium-m-sulfonatophenyl)phosphan in 30 ml Methanol gelöst vorgelegt (P/Rh-Molverhältnis: 60). Der Autoklav wird verschlossen, mit Wassergas gespült und auf eine Reaktionstemperatur von 120°C und einen Reaktionsdruck von 2 MPa eingestellt. Danach werden aus dem Dosiergefäß 10 g (50 mmol) 10-Undecensäuremethylester gegeben. Nach 1 h ist der Umsatz vollständig. Neben 3 % des Hydrierproduktes Undecansäuremethylester werden 97 % eines Gemisches aus 71 % 11- und 29 % 10-Formylundecansäuremethylester gebildet. Nach Abdestillieren des Methanols läßt sich das Katalysatorsystem in Wasser aufnehmen und von dem Reaktionsprodukt durch Phasenscheidung abtrennen.

### Beispiel 8

Beispiel 7 wird bei einem Reaktionsdruck von 1 MPa unter sonst identischen Bedingungen wiederholt. Bereits nach 30 min ist der Umsatz vollständig. Neben nur 1 % Undecansäuremethylester werden 99 % eines aus 70 Gew.-% 11- und 30 Gew.-% 10-Formylundecansäuremethylester bestehenden Gemisches erhalten. Das Katalysatorsystem wird nach Abdestillieren des Methanols in Wasser aufgenommen und vom Reaktionsprodukt abgetrennt.

### Beispiel 9

In ein Schlenkrohr werden unter Argon als Schutzgas 15 ml Methanol, 0,05 mmol Rh in Form von 60 mg einer 8,5 prozentigen Rhodiumlösung (als wäßrige Rh₂(SO₄)₃-Lösung) und 348 mg (1 mmol) Li-TPPMS gegeben (P/Rh-Molverhältnis: 20). Die Lösung wird auf einen pH-Wert von 6 eingestellt und unter Argon in einem Autoklav vorgelegt. Nach Schließen und Spülen des Autoklavs mit Wassergas stellt man einen Reaktionsdruck von 10 MPa und eine Reaktionstemperatur von 120°C ein. Aus einem Dosiergefäß setzt man 12 g (60 mmol) n-Tetradecen-1 zu. Nach 1 h ist das Olefin vollständig umgesetzt. Neben 2 % des Hydrierproduktes n-Tetradecan werden 98 % Hydroformylierungsprodukt der Zusammensetzung 72 Gew.-% n-Pentadecanal und 28 Gew.-% 2-Methyltetradecanal gebildet. Die Aldehyde fallen hauptsächlich (etwa 70 %) in Form der Dimethylacetale an. Das Reaktionsprodukt wird nach dem Abdestillieren der Hauptmenge des Lösungsmittels Methanol mit etwa 10 ml Wasser zur Abtrennung des Katalysatorkomplexes versetzt. Die organische Phase wird noch einmal mit etwa 5 ml Wasser extrahiert. Nach Vereinigung der wäßrigen Extrakte destilliert man das Wasser im Vakuum ab und löst das Katalysatorsystem wieder in Methanol.

### Beispiele 10 bis 15

Das in Beispiel 9 wie beschrieben zurückgewonnene Katalysatorsystem wird wiederholt zur Hydroformylierung von n-Tetradecen-1 eingesetzt. Die Bedingungen sind die gleichen wie in Beispiel 9. Die Ergebnisse der Versuche sind in Tabelle 2 zusammengestellt.

| Beispiel | Umsatz (%) | Ausbeute (%) | n-Pentadecanal (%) | 2-Methyltetradecanal (%) | Tetradecan (%) |
|---|---|---|---|---|---|
| 10 | 100 | 98 | 72 | 28 | 2 |
| 11 | 100 | 96 | 70 | 30 | 4 |
| 12 | 100 | 98 | 71 | 29 | 2 |
| 13 | 100 | 99 | 70 | 30 | 1 |
| 14 | 100 | 98 | 71 | 29 | 2 |
| 15 | 100 | 97 | 70 | 30 | 3 |

### Beispiel 16

In einem Schlenkrohr werden unter Argon als Schutzgas 25 ml Methanol, 0,05 mmol Rh in Form von 60 mg einer 8,5 prozentigen Rhodiumlösung (als wäßrige Rh₂(SO₄)₃-Lösung) und 348 mg (1 mmol) Li-TPPMS gegeben (P/Rh-Verhältnis: 20). Die Lösung wird auf einen pH-Wert von 11 eingestellt und unter Argon in einem Autoklav vorgelegt. Nach Schließen und Spülen des Autoklavs mit Wassergas stellt man einen Reaktionsdruck von 10 MPa und eine Reaktionstemperatur von 120°C ein. Aus einem Dosiergefäß setzt man 12 g (60 mmol) n-Tetradecen-1 zu. Nach 1 h ist das Olefin vollständig umgesetzt. Neben 2 % des Hydrierproduktes n-Tetradecen werden 98 % Hydroformylierungsprodukt der Zusammensetzung 71 Gew.-% n-Pentadecanal und 29 Gew.-% 2-Methyltetradecanal erhalten. Nur 3 % der gebildeten Aldehyde fallen (als Folge des hohen pH-Wertes 11) als Dimethylacetale an.

### Beispiel 17

In einem Schlenkrohr werden unter Argon als Schutzgas 15 ml Methanol, 0,05 mmol Rh in Form von 60 mg einer 8,5 prozentigen Rhodiumlösung (als Rh₂(SO₄)₃-Lösung) und 348 mg (1 mmol) Li-TPPMS gegeben (P/Rh-Verhältnis: 20). Die Lösung wird auf einen pH-Wert von 6 eingestellt und unter Argon in einem Autoklav vorgelegt. Nach Schließen und Spülen des Autoklavs mit Wassergas stellt man einen Reaktionsdruck von 20 MPa und eine Reaktionstemperatur von 120°C ein. Aus einem Dosiergefäß setzt man 16 g (50 mmol) technischen Linolensäuremethylester (55 % Linolensäuremethylester, Rest etwa 15 % Linolsäuremethylester, etwa 20 % Ölsäuremethylester) zu. Nach 3 h ist das Olefin vollständig umgesetzt. Das Reaktionsgemisch enthält 23 Gew.-% Monoformylprodukt, 22 Gew.-% Diformylprodukt und 47 Gew.-% Triformylprodukt. Bezogen auf den Linolensäuremethylesteranteil von 55 % im technischen Produkt bedeutet das eine Selektivität von 85 %, bezogen auf die Bildung des dreifach hydroformylierten Produktes.

## Patentansprüche

1. Verfahren zur Hydroformylierung olefinisch ungesättigter Verbindungen, deren Hydroformylierungsprodukte in Wasser nicht löslich sind, durch Umsetzung von olefinisch ungesättigten Verbindungen in homogener Phase in einem polaren organischen Lösungsmittel, bei dem es sich um einen niedermolekularen aliphatischen Alkohol mit 1 bis 4 Kohlenstoffatomen im Molekül oder um ein Gemisch aus zwei oder mehreren dieser Alkohole handelt und in Gegenwart eines Katalysatorsystems, das eine Rhodiumcarbonylverbindung und ein sowohl in dem polaren organischen Lösungsmittel als auch in Wasser löslichens Salz eines sulfonierten oder carboxylierten organischen Mono- oder Polyphosphins enthält, bei 60 bis 180°C und 1 bis 35 MPa mit Kohlenmonoxid und Wasserstoff, dadurch gekennnzeichnet, daß aus dem Reaktionsgemisch das polare organische Lösungsmittel abdestilliert und aus dem Destillationsrückstand das Katalysatorsystem mit Wasser abgetrennt wird.

2. Verfahren nach Anspruch 1, dadurch gekennzeichnet, daß die olefinisch ungesättigten Verbindungen acyclische Monoolefine mit zehn und mehr Kohlenstoffatomen im Molekül, mehrfach ungesättigte acyclische oder cyclische Olefine mit mindestens sechs Kohlenstoffatomen im Molekül oder Ester ungesättigter Fettsäuren sind.

3. Verfahren nach Anspruch 1 oder 2, dadurch gekennzeichnet, daß die olefinisch ungesättigten Verbindungen acyclische C₁₆- bis C₃₀-Monoolefine sind.

4. Verfahren nach Anspruch 1 oder 2, dadurch gekennzeichnet, daß die olefinisch ungesättigten Verbindungen mono- oder bicyclische Olefine mit mindestens sechs Kohlenstoffatomen im Molekül sind.

5. Verfahren nach Anspruch 1 oder 2, dadurch gekennzeichnet, daß die olefinisch ungesättigten Verbindungen Ester von zwei- oder dreifach ungesättigten Fettsäuren mit 8 bis 25 Kohlenstoffatomen im Molekül sind.

6. Verfahren nach Anspruch 5, dadurch gekennzeichnet, daß sich die Ester von zwei- oder dreifach ungesättigten Fettsäuren mit 10 bis 20 Kohlenstoffatomen im Molekül ableiten.

7. Verfahren nach einem oder mehreren der Ansprüche 2, 5 und 6, dadurch gekennzeichnet, daß sich die Ester von gesättigten aliphatischen Monoalkoholen mit 1 bis 10 Kohlenstoffatomen im Molekül ableiten.

8. Verfahren nach Anspruch 7, dadurch gekennzeichnet, daß der aliphatische Monoalkohol Methanol ist.

9. Verfahren nach Anspruch 1, dadurch gekennzeichnet, daß das polare organische Lösungsmittel Methanol oder Ethanol ist.

10. Verfahren nach einem oder mehreren der Ansprüche 1 bis 9, dadurch gekennzeichnet, daß das Kation des in einem polaren organischen Lösungsmittel und in Wasser löslichen Salzes des sulfonierten oder carboxylierten organischen Phosphins Lithium oder ein Ammonium-Ion der allgemeinen Formel N(R¹R²R³R⁴)⁺ ist, in der R¹, R², R³ und R⁴ gleich oder verschieden sind und für Wasserstoff und geradkettige oder verzweigte Alkylreste, insbesondere Alkylresten mit 1 bis 4 Kohlenstoffatomen stehen.

11. Verfahren nach einem oder mehreren der Ansprüche 1 bis 10, dadurch gekennzeichnet, daß das Anion des in Wasser und in einem polaren organischen Lösungsmittel löslichen Salzes des sulfonierten oder carboxylierten organischen Phosphins der allgemeinen Formel 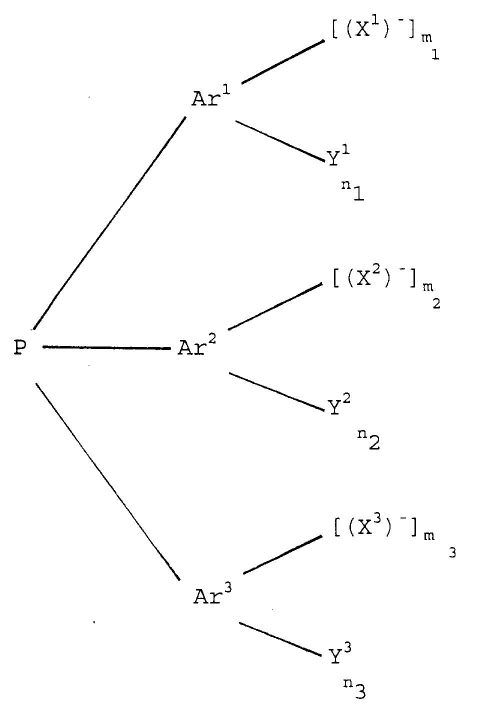 entspricht, in der Ar¹, Ar², Ar³ jeweils eine Phnyl- oder Naphthylgruppe bedeutet, X¹, X², X³ jeweils für einen Sulfonat-(-SO₃⁻) und/oder einen Carboxylat-(-COO⁻) Rest steht, Y¹, Y², Y³ jeweils eine geradkettige oder verzweigte Alkylgruppe mit 1 bis 4 C-Atomen, eine Alkoxygruppe, ein Halogenatom, die OH-, CN-, NO₂- oder die (R⁵R⁶)-N-Gruppe, in der R⁵ und R⁶ für jeweils eine geradkettige oder verzweigte Alkylgruppe mit 1 bis 4 C-Atomen stehen, ist, m₁, m₂, m₃ gleiche oder verschiedene ganze Zahlen von 0 bis 5 sind mit der Maßgabe, daß mindestens ein m₁, m₂ oder m₃ größer als 0 ist und n₁, n₂, n₃ gleiche oder verschiedene ganze Zahlen von 0 bis 5 wiedergeben.

12. Verfahren nach Anspruch 11, dadurch gekennzeichnet, daß das in einem polaren organischen Lösungsmittel und in Wasser lösliche Salz des sulfonierten organischen Phosphins das Tris(m-sulfonatophenyl)phosphin-Anion enthält.

13. Verfahren nach einem oder mehreren der Ansprüche 1 bis 12, dadurch gekennzeichnet, daß das Anion des in einem polaren organischen Lösungsmittel und in Wasser löslichen Salzes des sulfonierten organischen Phosphins der allgemeinen Formel
A₂P-(CH₂)ₘ-CH(R)-SO₃⁻
entspricht, in der A gleiche oder verschiedene Alkyl- und/oder Arylreste bedeutet, n 0, 1 oder 2 ist und R für Wasserstoff oder einen Alkylrest steht.

14. Verfahren nach einem oder mehreren der Ansprüche 1 bis 10, dadurch gekennzeichnet, daß das Anion des in einem polaren organischen Lösungsmittel und in Wasser löslichen Salzes sich von Diarylverbindungen der allgemeinen Formel 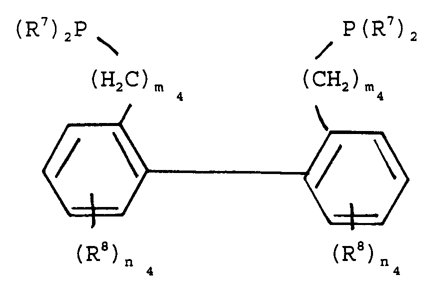 ableiten, die durch mindestens einen Sulfonatrest (-SO₃⁻) oder einen Carboxylatrest (-COO⁻) substituiert sind und in der R⁷ für gleiche oder verschiedene Alkyl-, Cycloalkyl-, Phenyl-, Tolyl- oder Napthylreste steht, R⁸ gleich oder verschieden ist und Wasserstoff, Alkyl- oder Alkoxyreste mit 1 bis 14 C-Atomen, ferner Cycloalkyl-, Aryl- oder Aroxyreste mit 6 bis 14 C-Atomen oder einen annelierten Benzolring bedeutet, m₄ gleich oder verschieden ist und für ganze Zahlen von 0 bis 5 steht und n₄ ebenfalls gleich oder verschieden ist und für ganze Zahlen von 0 bis 4 steht.

15. Verfahren nach Anspruch 14, dadurch gekennzeichnet, daß sich das Anion des in einem polaren organischen Lösungsmittel und in Wasser löslichen Salzes von Diarylverbindungen ableitet, die durch mindestens einen Sulfonatrest substituiert sind.

16. Verfahren nach einem oder mehreren der Ansprüche 1 bis 15, dadurch gekennzeichnet, daß die Umsetzung der olefinisch ungesättigten Verbindungen mit Wasserstoff und Kohlenmonoxid bei 100 bis 140°C erfolgt.

17. Verfahren nach einem oder mehreren der Ansprüche 1 bis 4 und 10 bis 16, dadurch gekennzeichnet, daß die Umsetzung von Olefinen mit Wasserstoff und Kohlenmonoxid bei Drücken von 2 bis 35 MPa erfolgt.

18. Verfahren nach einem oder mehreren der Ansprüche 1, 2 und 5 bis 16, dadurch gekennzeichnet, daß die Umsetzung der ungesättigten Fettsäureester mit Kohlenmonoxid und Wasserstoff bei 15 bis 25 MPa erfolgt.

19. Verfahren nach einem oder mehreren der Ansprüche 1 bis 18, dadurch gekennzeichnet, daß die Rhodiumkonzentration in der Katalysatorlösung 100 bis 600 Gew.-ppm, bezogen auf die Lösung, beträgt.

20. Verfahren nach Anspruch 19, dadurch gekennzeichnet, daß die Rhodium-Konzentration in der Reaktionslösung 300 bis 400 Gew.-ppm, bezogen auf die Lösung, beträgt.

21. Verfahren nach einem oder mehreren der Ansprüche 1 bis 20, dadurch gekennzeichnet, daß in der Reaktionslösung je mol Rhodium mindestens 20 mol, vorzugsweise 40 bis 80 mol, P(III) vorliegen.

22. Verfahren nach einem oder mehreren der Ansprüche 1 bis 21, dadurch gekennzeichnet, daß der pH-Wert der Reaktionslösung mindestens 3, vorzugsweise 4 bis 11, ist.

23. Verfahren nach einem oder mehreren der Ansprüche 1 bis 8 und 10 bis 21, dadurch gekennzeichnet, daß der pH-Wert der Reaktionslösung 4,5 bis 6,5, insbesondere 5,5 bis 6,0, ist.

24. Verfahren nach einem oder mehreren der Ansprüche 1 bis 23, dadurch gekennzeichnet, daß die Abtrennung des Katalysatorsystems aus dem Destillationsrückstand durch mehrfache Behandlung mit Wasser erfolgt.

25. Verfahren nach einem oder mehreren der Ansprüche 1 bis 24, dadurch gekennzeichnet, daß dem Wasser zur Behandlung des Destillationsrückstandes ein zur Komplexbildung mit Rhodium befähigtes Phosphin zugesetzt wird.

## Claims

1. A process for the hydroformylation of olefinically unsaturated compounds whose hydroformylation products are insoluble in water by reacting olefinically unsaturated compounds at 60 to 180°C and 1 to 35 MPa with carbon monoxide and hydrogen in a homogeneous phase in a polar organic solvent which is a low-molecular aliphatic alcohol having 1 to 4 carbon atoms in the molecule or a mixture of two or more of said alcohols and in the presence of a catalyst system comprising a rhodium carbonyl compound and a salt of a sulfonated or carboxylated organic monophosphine or polyphosphine, which salt is soluble both in the polar organic solvent and in water, characterised in that the polar organic solvent is distilled off from the reaction mixture and the catalyst system is separated from the distillation residue using water.

2. The process according to claim 1, characterised in that the olefinically unsaturated compounds are acyclic monoolefins having ten or more carbon atoms in the molecule, polyunsaturated acyclic or cyclic olefins having at least six carbon atoms in the molecule or esters of unsaturated fatty acids.

3. The process according to claim 1 or 2, characterised in that the olefinically unsaturated compounds are acyclic C₁₆ to C₃₀ monoolefins

4. The process according to claim 1 or 2, characterised in that the olefinically unsaturated compounds are monocyclic or bicyclic olefins having at least six carbon atoms in the molecule.

5. The process according to claim 1 or 2, characterised in that the olefinically unsaturated compounds are esters of doubly or triply unsaturated fatty acids having 8 to 25 carbon atoms in the molecule.

6. The process according to claim 5, characterised in that the esters are derived from doubly or triply unsaturated fatty acids having 10 to 20 carbon atoms in the molecule.

7. The process according to one or more of claims 2, 5 and 6, characterised in that the esters are derived from saturated aliphatic monoalcohols having 1 to 10 carbon atoms in the molecule.

8. The process according to claim 7, characterised in that the aliphatic monoalcohol is methanol.

9. The process according to claim 1, characterised in that the polar organic solvent is methanol or ethanol.

10. The process according to one or more of claims 1 through 9, characterised in that the cation of the salt of the sulfonated or carboxylated organic phosphine, which salt is soluble in a polar organic solvent and in water, is lithium or an ammonium ion of the formula N(R¹R²R³R⁴)⁺ in which R¹, R², R³ and R⁴ are identical or different and are hydrogen or straight-chain or branched alkyl radicals, in particular alkyl radicals having 1 to 4 carbon atoms.

11. The process according to one or more of claims 1 to 10, characterised in that the anion of the salt of the sulfonated or carboxylated organic phosphine, which salt is soluble in water and in a polar organic solvent, has the formula 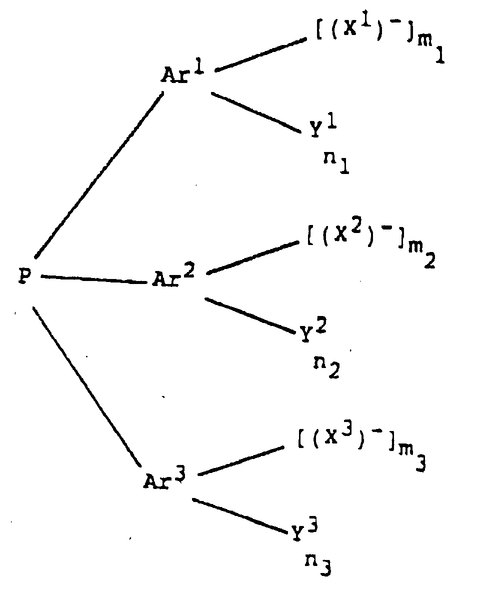 where Ar¹, Ar², Ar³ are each a phenyl or naphthyl group, X¹, X², X³ are each a sulfonate (-SO₃⁻) and/or a carboxylate (-COO⁻) radical, Y¹, Y², Y³ are each a straight-chain or branched alkyl group having 1 to 4 carbon atoms, an alkoxy group, a halogen atom, the OH, CN, NO₂ or (R⁵R⁶)-N group in which R⁵ and R⁶ are each a straight-chain or branched alkyl group having 1 to 4 carbon atoms, m₁, m₂, m₃ are identical or different integers from 0 to 5, with the proviso that at least one m₁, m₂ or m₃ is greater than 0 and n₁, n₂, n₃ are identical or different integers from 0 to 5.

12. The process according to claim 11, characterised in that the salt of the sulfonated organic phosphine, which salt is soluble in a polar organic solvent and in water, contains the tris(m-sulfonatophenyl)phosphine anion.

13. The process according to one or more of claims 1 through 11, characterised in that the anion of the salt of the sulfonated organic phosphine, which salt is soluble in a polar organic solvent and in water, has the formula
A₂P-(CH₂)ₘ-CH(R)-SO₃
where A is identical or different alkyl and/or aryl radicals, n is 0, 1 or 2 and R is hydrogen or an alkyl radical

14. The process according to one or more of claims 1 through 10, characterised in that the anion of the salt which is soluble in a polar organic solvent and in water is derived from diaryl compounds of the formula 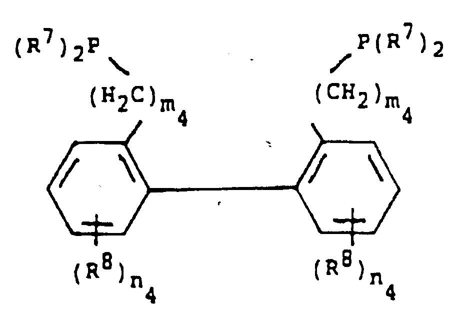 which are substituted by at least one sulfonate radical (-SO₃⁻) or carboxylate radical (-COO⁻), where R⁷ stands for identical or different alkyl, cycloalkyl, phenyl, tolyl or naphthyl radicals, R⁸ is identical or different and is hydrogen, alkyl or alkoxy radicals having 1 to 14 carbon atoms, also cycloalkyl, aryl or aroxy radicals having 6 to 14 carbon atoms or a fused-on benzene ring, m₄ is identical or different and denotes integers from 0 to 5 and n₄ is likewise identical or different and denotes integers from 0 to 4.

15. The process according to claim 14, characterised in that the anion of the salt which is soluble in a polar organic solvent and in water is derived from diaryl compounds which are substituted by at least one sulfonate radical.

16. The process according to one or more of claims 1 through 15, characterised in that the reaction of the olefinically unsaturated compounds with hydrogen and carbon monoxide is carried out at 100 to 140°C.

17. The process according to one or more of claims 1 through 4 and 10 through 16, characterised in that the reaction of olefins with hydrogen and carbon monoxide is carried out at pressures of 2 to 35 MPa.

18. The process according to one or more of claims 1, 2 and 5 through 16, characterised in that the reaction of the unsaturated fatty acid esters with carbon monoxide and hydrogen is carried out at 15 to 25 MPa.

19. The process according to one or more of claims 1 through 18, characterised in that the rhodium concentration in the catalyst solution is 100 to 600 ppm by weight, based on the solution.

20. The process according to claim 19, characterised in that the rhodium concentration in the reaction solution is 300 to 400 ppm by weight, based on the solution.

21. The process according to one or more of claims 1 to 20, characterised in that at least 20 mol, preferably from 40 to 80 mol, of P(III) are present in the reaction solution per mol of rhodium.

22. The process according to one or more of claims 1 to 21, characterised in that the pH of the reaction solution is at least 3, preferably 4 to 11.

23. The process according to one or more of claims 1 to 8 and 10 to 21, characterised in that the pH of the reaction solution is 4.5 to 6.5, in particular 5.5 to 6.0.

24. The process according to one or more of claims 1 to 23, characterised in that the catalyst system is separated from the distillation residue by a multiple treatment with water.

25. The process according to one or more of claims 1 to 24, characterised in that a phosphine capable of complex formation with rhodium is added to the water for treating the distillation residue.

## Revendications

1. Procédé d'hydroformylation de composés oléfiniquement insaturés dont les produits d'hydroformylation ne sont pas solubles dans l'eau, par conversion de composés oléfiniquement insaturés en phase homogène dans un solvant organique polaire qui est un alcool aliphatique de faible masse moléculaire ayant 1 à 4 atomes de carbone dans la molécule ou un mélange de 2 ou plusieurs de ces alcools et en présence d'un système catalytique qui contient un composé de type rhodiumcarbonyle et un sel soluble aussi bien dans le solvant organique polaire que dans l'eau d'une mono- ou polyphosphine organique sulfonée ou carboxylée, entre 60 et 180°C et 1 et 35 MPa avec du monoxyde de carbone et de l'hydrogène, caractérisé en ce que le solvant organique polaire est retiré du mélange réactionnel par distillation et le système catalytique est séparé du résidu de distillation avec de l'eau.

2. Procédé selon la revendication 1, caractérisé en ce que les composés oléfiniquement insaturés sont des monooléfines acycliques ayant 10 atomes de carbone et plus dans la molécule, des oléfines acycliques ou cycliques plusieurs fois insaturées ayant au moins six atomes de carbone dans la molécule ou des esters d'acides gras insaturés.

3. Procédé selon la revendication 1 ou 2 caractérisé en ce que les composés oléfiniquement insaturés sont des monooléfines acycliques en C₁₆ à C₃₀.

4. Procédé selon la revendication 1 ou 2, caractérisé en ce que les composés oléfiniquement insaturés sont des oléfines mono- ou bicycliques ayant au moins six atomes de carbone dans la molécule.

5. Procédé selon la revendication 1 ou 2, caractérisé en ce que les composés oléfiniquement insaturés sont des esters d'acides gras deux ou trois fois insaturés ayant 8 à 25 atomes de carbone dans la molécule.

6. Procédé selon la revendication 5, caractérisé en ce que les esters dérivent d'acides gras deux ou trois fois insaturés ayant 10 à 20 atomes de carbone dans la molécule.

7. Procédé selon une ou plusieurs des revendications 2, 5 et 6, caractérisé en ce que les esters dérivent de monoalcools aliphatiques saturés ayant 1 à 10 atomes de carbone dans la molécule.

8. Procédé selon la revendication 7, caractérisé en ce que le monoalcool aliphatique est le méthanol.

9. Procédé selon la revendication 1, caractérisé en ce que le solvant organique polaire est le méthanol ou l'éthanol.

10. Procédé selon une ou plusieurs des revendications 1 à 9, caractérisé en ce que le cation du sel soluble dans un solvant organique polaire et dans l'eau de la phosphine organique sulfonée ou carboxylée est le lithium ou un ion ammonium de formule générale N(R¹R²R³R⁴)⁺ dans la laquelle R¹, R², R³ et R⁴ sont identiques ou différents et représentent l'hydrogène ou des restes alkyle linéaires ou ramifiés, en particulier des restes alkyle ayant 1 à 4 atomes de carbone.

11. Procédé selon une ou plusieurs des revendications 1 à 10, caractérisé en ce que l'anion du sel soluble dans l'eau et dans un solvant organique polaire de la phosphine organique sulfonée ou carboxylée correspond à la formule générale 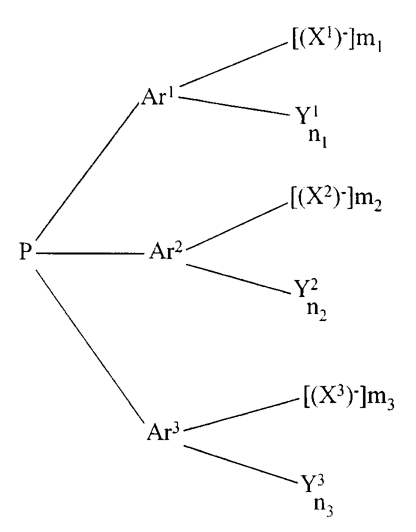 dans laquelle Ar¹, Ar², Ar³ représentent chacun un groupe phényle ou naphtyle, X¹, X², X³ représentent chacun un reste sulfonate (-SO₃⁻) et/ou un reste carboxylate (-COO⁻), Y¹, Y², Y³ représentent chacun un groupe alkyle linéaire ou ramifié ayant 1 à 4 atomes de carbone, un groupe alcoxy, un atome d'halogène, le groupe OH-, CN-, NO₂- ou (R⁵R⁶)-N-, dans lequel R⁵ et R⁶ représentent chacun un groupe alkyle linéaire ou ramifié ayant 1 à 4 atomes de carbone, m₁, m₂, m₃ sont des nombres entiers identiques ou différents, de 0 à 5, à condition qu'au moins un m₁, m₂ ou m₃ soit supérieur à 0 et n₁, n₂, n₃ représentent des nombres entiers identiques ou différents, de 0 à 5.

12. Procédé selon la revendication 11, caractérisé en ce que le sel soluble dans un solvant organique polaire et dans l'eau de la phosphine organique sulfonée contient l'anion tris(m-sulfonatophényl)phosphine.

13. Procédé selon une ou plusieurs des revendications 1 à 12, caractérisé en ce que l'anion du sel soluble dans un solvant organique polaire et dans l'eau de la phosphine organique sulfonée correspond à la formule générale
A₂P-(CH₂)ₘ-CH(R)-SO₃⁻
dans laquelle A représente des restes alkyle et/ou aryle identiques ou différents, n est 0, 1 ou 2 et R représente l'hydrogène ou un reste alkyle.

14. Procédé selon une ou plusieurs des revendications 1 à 10, caractérisé en ce que l'anion du sel soluble dans un solvant organique polaire et dans l'eau dérive de composés diaryle de formule générale 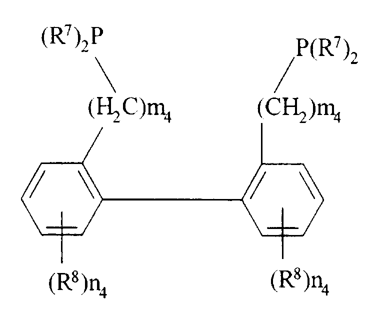 qui sont substitués par au moins un reste sulfonate (-SO₃⁻) ou un reste carboxylate (-COO⁻) et dans lesquels les R⁷ représentent des restes alkyle, cycloalkyle, phényle, tolyle ou naphtyle identiques ou différents, les R⁸ sont identiques ou différents et représentent l'hydrogène, des restes alkyle ou alcoxy ayant 1 à 14 atomes de carbone, en outre des restes cycloalkyle, aryle ou aryloxy ayant 6 à 14 atomes de carbone ou un cycle benzénique condensé, les m₄ sont identiques ou différents et représentent des nombres entiers de 0 à 5 et les n₄ sont de même identiques ou différents et représentent des nombres entiers de 0 à 4.

15. Procédé selon la revendication 14, caractérisé en ce que l'anion du sel soluble dans un solvant organique polaire et dans l'eau dérive de composés diaryle qui sont substitués par au moins un reste sulfonate.

16. Procédé selon une ou plusieurs des revendications 1 à 15, caractérisé en ce que la conversion des composés oléfiniquement insaturés avec l'hydrogène et le monoxyde de carbone a lieu entre 100 et 140°C.

17. Procédé selon une ou plusieurs des revendications 1 à 4 et 10 à 16, caractérisé en ce que la conversion des oléfines avec l'hydrogène et le monoxyde de carbone a lieu à des pressions de 2 à 35 MPa.

18. Procédé selon une ou plusieurs des revendications 1, 2 et 5 à 16, caractérisé en ce que la conversion des esters d'acides gras insaturés avec le monoxyde de carbone et l'hydrogène a lieu entre 15 et 25 MPa.

19. Procédé selon une ou plusieurs des revendications 1 à 18, caractérisé en ce que la concentration du rhodium dans la solution de catalyseur est de 100 à 600 ppm en masse par rapport à la solution.

20. Procédé selon la revendication 19, caractérisé en ce que la concentration du rhodium dans la solution réactionnelle est de 300 à 400 ppm en masse par rapport à la solution.

21. Procédé selon une ou plusieurs des revendications 1 à 20, caractérisé en ce qu'il y a au moins 20 mol, de préférence 40 à 80 mol, de P(III) par mole de rhodium dans la solution réactionnelle.

22. Procédé selon une ou plusieurs des revendications 1 à 21, caractérisé en ce que le pH de la solution réactionnelle est d'au moins 3, de préférence de 4 à 11.

23. Procédé selon une ou plusieurs des revendications 1 à 8 et 10 à 21. caractérisé en ce que le pH de la solution réactionnelle est de 4,5 à 6,5, en particulier de 5,5 à 6,0.

24. Procédé selon une ou plusieurs des revendications 1 à 23, caractérisé en ce que la séparation du système catalytique à partir du résidu de distillation a lieu par traitement répété à l'eau.

25. Procédé selon une ou plusieurs des revendications 1 à 24, caractérisé en ce qu'une phosphine capable de se complexer avec le rhodium est ajoutée à l'eau pour le traitement du résidu de distillation.
